# NEUE EUROPÄISCHE PATENTSCHRIFT

(11) **EP 1 904 029 B2**
(45) Veröffentlichungstag und Bekanntmachung des Hinweises auf die Entscheidung über den Einspruch: **12.11.2014**
(45) Hinweis auf die Patenterteilung: 26.01.2011
(21) Anmeldenummer: 06776333.4
(22) Anmeldetag: 20.07.2006
(51) Int. Cl.: A61K 9/00, A61K 9/70, A61K 31/551, A61P 25/18

(54) **NICHT-AUSSPUCKBARER, ORALER, SCHNELL-ZERFALLENDER FILM ENTHALTEND OLANZAPIN**
ORAL, QUICKLY DISINTEGRATING FILM, WHICH CANNOT BE SPIT OUT, CONTAINING OLANZAPIN
FILM A DECOMPOSITION RAPIDE, QUI EST ORAL ET NE DEGORGE PAS COMPRENANT DE L'OLANZAPINE

(30) Priorität: 20.07.2005 DE 102005033943
(43) Veröffentlichungstag der Anmeldung: 02.04.2008
(73) Patentinhaber: Hexal AG, 83607 Holzkirchen (DE)
(72) Erfinder: OBERMEIER, Petra, 81673 München (DE); KOHR, Thomas, 81675 München (DE); KRAMER, Kai-Thomas, 83646 Bad Tölz (DE); KLOKKERS, Karin, 83677 Reichersbeuern (DE)
(74) Vertreter: Weiss, Wolfgang
(86) Internationale Anmeldenummer: PCT/EP2006/007177
(87) Internationale Veröffentlichungsnummer: WO 2007/009801

(56) Entgegenhaltungen:
- EP-A1- 0 441 333
- EP-A2- 0 180 364
- WO-A-00/45798
- WO-A-01/30288
- WO-A-01/70194
- WO-A-2004/091585
- WO-A-2004/096193
- US-A1- 2004 247 649
- US-A1- 2005 118 217

## Beschreibung

Die Erfindung bezieht sich auf einen nicht-ausspuckbaren, oralen, schnell zerfallenden, einschichtigen Film mit einem Neuroleptikum, dessen Herstellung und dessen Verwendung. Dabei wird Olanzapin als Neuroleptikum eingesetzt.

Pharmazeutische Darreichungsformen wie beispielsweise Schmelztabletten, die im Mund kleben und schnell zerfallen, sind in vielerlei Hinsicht vorteilhaft. Sie erleichtern die orale Verabreichung von Medikamenten an Patienten mit psychischen Erkrankungen wie Schizophrenie, die einer Therapie mit anderen oralen Arzneiformen (z.B. Filmtabletten) nur schwer zugänglich sind. Aufgrund der Mucoadhesivität und des schnellen Zerfalls der Darreichungsform ist es dem Patienten nicht möglich, die Arzneiform z.B. in der Mundhöhle aufzubewahren und später wieder auszuspucken. Nachteilig an Schmelztabletten ist jedoch deren kostenintensive Herstellung, die einen aufwendigen Lyophilisierungsprozeß erfordert; vgl. z.B. DE 27 44 493, EP 0 793 495 und WO 01/39 836. Darüber hinaus sind einige Wirkstoffe, wie z.B. Olanzapin, in Filmtabletten nur bedingt chemisch stabil.

Als orale Arzneiformen, welche mucoadhesiv sind und oral schnell zerfallen, kommen auch flächenförmige Filme in Betracht. Diese zeichnen sich durch eine geringe Schichtdicke und somit durch eine große Oberfläche aus, was einen schnellen Zerfall bewirkt.

So werden in EP 936905 mucoadhesive Filme mit Hypnotica, Antiepileptika oder Psychoneurotropika beschrieben, die ein Tensid enthalten. Nachteilig an der Verwendung von Tensiden ist jedoch deren mögliche haut- bzw. schleimhautreizende Wirkung. Ausserdem schmecken viele der üblichen Tenside sehr bitter. Nachteilig ist ebenfalls eine mögliche Interaktion bei der Wirkstoffaufnahme im Gastrointestinal-Trakt.

WO 03/101 420 beschreibt Filme mit verringerter Tendenz zur Mundschleimhaut-Haftung, WO 03/070 227 mucoadhäsive Filme, und zwar jeweils Filme z.B. mit Psychopharmaka, die einen Kohlendioxidbildner als Brausezusatz enthalten. Nachteilig an einem Brausezusatz sind der saure Geschmack sowie die Schaumbildung im Mund. Zudem ist die Formulierung sehr feuchteempfindlich. Auch eine mögliche chemische Interaktion der Brausebestandteile mit den Hilfsstoffen der Formulierung ist nachteilig.

WO 02/02085 offenbart Filme mit verringerter Tendenz zur Mundschleimhaut-Haftung und mit Hohlräumen, um die Haftung des Films an der Mundschleimhaut zu verringern.

In WO 01/70194 und US 20040247649 werden mucoadhäsive Filme mit wasserlöslichen Polymeren, einem Geschmacksmaskierer und Wirkstoffen, z.B. Psychopharmaka, beschrieben.

Aufgabe der Erfindung ist die Bereitstellung eines nicht-ausspuckbaren Films mit einem Neuroleptikum, insbesondere Olanzapin. Der Film soll für die orale Verabreichung des Neuroleptikums geeignet sein. Der Film soll im Mund nach Kontakt mit Flüssigkeit oder Speichel kleben und dort schnell zerfallen, beispielsweise unter Einwirkung von Speichel aufgelöst oder zersetzt werden. Der wirkstoffhaltige Film soll sowohl chemisch als auch physikalisch stabil sein. Der Film soll frei von den oben genannten Tensiden, Brausezusätzen oder Geschmacksmaskierern sein. Die Herstellung des Films soll kostengünstig sein.

Zur Lösung der Aufgabe stellt die Erfindung eine filmförmige Zubereitung zur oralen Applikation bereit, umfassend einen oder mehrere Filmbildner, ausgewählt aus der durch Ethylcellulose, Celluloseacetat, Cellulosephthalat, Sorbit, Xylit, Polyethylenglykol, 1,3-Butandiol, Propylenglykol, Isopropylpalmitat, Dibutylsebacat und Paraffinöl gebildeten Gruppe; wobei mindestens ein Filmbildner wasserunlöslich ist und ausgewählt ist aus der Gruppe wasserunlösliche Ethylcellulose, wasserunlösliches Celluloseacetat, wasserunlösliches Cellulosephthalat und Paraffinöl,
einen oder mehrere Gelbildner, wobei der Gelbildner ein Cellulosederivat mit einem Molekulargewicht von weniger als 60000 Dalton ist und Hydroxypropylcellulose und/oder Hydroxypropylmethylcellulose umfasst, und
Olanzapin. Die filmförmige Zubereitung ist bevorzugt einschichtig und vorzugsweise im wesentlichen frei von Hohlräumen, Tensiden, Brausezusätzen und Geschmacksmaskierern. Bevorzugt ist die filmförmige Zubereitung ein Film, insbesondere ein fester Film. Bevorzugt ist der Film einschichtig. Bevorzugt ist der Film im wesentlichen frei von Hohlräumen, Tensiden, Brausezusatz und Geschmacksmaskierern. Bevorzugt zerfällt der Film rasch in Speichel.

Es wurde gefunden, dass die erfindungsgemäße Zubereitung eine sehr vorteilhafte Kombination aus mechanischer Stabilität des Films und schneller Freisetzung des Wirkstoffes bietet.

So betrifft eine Ausführungsform der Erfindung eine einschichtige filmförmige Zubereitung, wie in den Ansprüchen definiert. Bevorzugt ist die filmförmige Zubereitung im wesentlichen frei von Hohlräumen, Tensiden, Brausezusatz und Geschmacksmaskierern.

Dabei bedeutet der Begriff einschichtige filmförmige Zubereitung vorzugsweise eine feste Zubereitung, die in Form eines einschichtigen Films vorliegt. Dabei bedeutet einschichtig, dass der Film in Form einer einzigen Schicht vorliegt, wobei die Schicht bevorzugt homogen ist. Der Film kann flexibel oder nicht flexibel sein, ist aber vorzugsweise flexibel.

Bevorzugt ist die einschichtige filmförmige Zubereitung im wesentlichen frei von Hohlräumen. Dabei wird unter einem Hohlraum ein Bereich verstanden, der mit einem Fluid (einem Gas und/oder einer Flüssigkeit) gefüllt ist. Ein solcher Hohlraum hat üblicherweise einen Durchmesser von weniger als 100 µm. Bevorzugt ist eine filmförmige Zubereitung im wesentlichen frei von Gasblasen und/oder Hohlräumen, die ein Fluid (Gas und/oder Flüssigkeit) enthalten.

Bevorzugt ist die einschichtige filmförmige Zubereitung im wesentlichen frei von Tensiden. Dabei bedeutet im wesentlichen frei von Tensiden, dass die filmförmige Zubereitung, bezogen auf die gesamte Zubereitung, weniger als 1 Gew.-%, bezogen auf die getrocknete Zubereitung, bevorzugt weniger als 0,1 Gew.-% und besonders bevorzugt weniger als 0,01 Gew.-% Tensid enthält. Insbesondere werden keine Tenside bei der Herstellung der filmförmigen Zubereitung als Bestandteil zugesetzt. Ein Tensid im Sinne dieser Erfindung ist jedes übliche Tensid, Netzmittel oder oberflächenaktive Substanz.

Bevorzugt ist die einschichtige filmförmige Zubereitung im wesentlichen frei von Brausezusatz. Dabei bedeutet im wesentlichen frei von Brausezusatz, dass die filmförmige Zubereitung, bezogen auf die gesamte Zubereitung, weniger als 1 Ges.-%, bezogen auf die getrocknete Zubereitung, bevorzugt weniger als 0,1 Gew.-% und besonders bevorzugt weniger als 0,01 Ges.-% Brausezusatz enthält. Insbesondere wird kein Brausezusatz bei der Herstellung der filmförmigen Zubereitung als Bestandteil zugesetzt. Ein Brausezusatz im Sinne dieser Erfindung ist eine Verbindung, die bei Zusatz von Wasser, Lagerung, erhöhter Temperatur oder dergleichen eine gasförmige Verbindung freisetzt. Bevorzugt ist ein Brausezusatz eine Verbindung, die im Mund, z.B. durch Einwirkung von Speichel, eine gasförmige Verbindung freisetzt, wie beispielsweise ein Kohlendioxidbildner. Die filmförmige Zubereitung enthält also keinen oder nahezu keinen Brausezusatz, wie beispielweise einen Kohlendioxidbildner.

Bevorzugt ist die einschichtige filmförmige Zubereitung im wesentlichen frei von Geschmacksmaskierern. Dabei bedeutet im wesentlichen frei von Geschmacksmaskierern, dass die filmförmige Zubereitung, bezogen auf die gesamte Zubereitung, weniger als 1 Gew.-%, bezogen auf die getrocknete Zubereitung, bevorzugt weniger als 0,1 Gew.-% und.besonders bevorzugt weniger als 0,01 Gew.-% Geschmacksmaskierer enthält. Insbesondere werden keine Geschmacksmaskierer bei der Herstellung der filmförmigen Zubereitung als Bestandteil zugesetzt. Ein Geschmacksmaskierer im Sinne dieser Erfindung interagiert mit einer schlecht schmeckenden Substanz, wodurch deren schlechter Geschmack "maskiert" wird.

Unter einem Geschmacksmaskierer versteht man insbesondere eine Substanz, die zur Überdeckung des schlechten Geschmacks, beispielsweise eines Wirkstoffs dient. Der Film bzw. die filmförmige Zubereitung ist insbesondere frei von Mischungen des Wirkstoffs mit Ionenaustauscherharzen, Einschlußverbindungen des Wirkstoffs mit Cyclodextrin oder Beschichtungen des Wirkstoffs mit einem Überzug, z.B. Eudragit. Bevorzugt ist der Wirkstoff in freier Form in der Zubereitung enthalten, und nicht beispielsweise verkapselt oder abgeschlossen.

Eine weitere Ausführungsform betrifft filmförmige, einschichtige und vorzugsweise hohlraumfreie Zubereitungen frei von Tensiden, Brausezusatz und Geschmacksmaskierern aus einem oder mehreren Filmbildnern, einem oder mehreren Gelbildnern und Olanzapin.

Die erfindungsgemäße Zubereitung ist frei von Geschmacksmaskieren, kann jedoch fakultativ Süßungsmittel oder Geschmacksstoffe enthalten.

Bei der erfindungsgemäßen Zubereitung kann der Wirkstoffgehalt im Film 0,1 bis 60 Gew. % und insbesondere bis 50 Gew.-% und vorzugsweise 20 bis 30 Gew.-% und besonders bevorzugt etwa 25 Gew.-% betragen, jeweils bezogen auf die getrocknete Zubereitung.

Erfindungsgemäß wird wasserunlöslich vorzugsweise so definiert, dass 1 Teil einer Verbindung (1 Teil des Filmbildners bzw. des Gelbindners) insbesondere gemäß dem Deutschen Arzneibuch (9. Ausgabe vom 1. 7. 1987) in 30 bis 100 Teilen Wasser, stärker bevorzugt in 100 bis 1000 Teilen Wasser, stärker bevorzugt in 1000 bis 10000 Teilen Wasser und besonders bevorzugt in mehr als 10000 Teilen Wasser löslich ist. Wasserlöslich wird vorzugsweise so definiert, dass 1 Teil einer Verbindung (1 Teil des Filmbildners bzw. des Gelbildners) insbesondere gemäß dem Deutschen Arzneibuch (9. Ausgabe vom 1. 7. 1987) in 10 bis 30 Teilen Wasser, stärker bevorzugt in 1 bis 10 Teilen und besonders bevorzugt in weniger als 1 Teilen Wasser löslich ist.

Bei der erfindungsgemäßen Zubereitung kann der Gehalt des Films an Filmbildner 5 bis 70 Gew.-% betragen, bevorzugt 5 bis 30 Gew.-%, jeweils bezogen auf die getrocknete Zubereitung.

Ein Filmbildner im Sinne dieser Erfindung ist insbesondere eine Verbindung, die der Filmzubereitung eine gewisse Flexibilität der mechanischen Eigenschaften, wie beispielsweise Rückstellkraft, Biegemodul, Dehnungsmodul und dergleichen, verleiht.

Bei der erfindungsgemäßen Zubereitung kann der Gehalt des Films an Gelbildner 10 bis 70 Gew.-% betragen, bevorzugt 20 bis 50 Gew.-%, jeweils bezogen auf die getrocknete Zubereitung.

Polymere Verbindungen mit einem Molekulargewicht von weniger als 60000 Dalton, vorzugsweise von 10000 bis 40000 Dalton begünstigen vorteilhaft einen schnellen Zerfall der Zubereitung.

Für die erfindungsgemäße Zubereitung wird eine Kombination aus mindestens zwei Gelbildnern bevorzugt; gemäß einer weiteren Ausführungsform ist einer der Gelbildner wasserunlöslich.

Besonders bevorzugt ist eine Kombination aus wasserunlöslicher Ethylcellulose und Hydroxypropylcellulose mit einem Molukargewicht von weniger als 60000 Dalton und/oder Hydroxypropylmethylcellulose mit einem Molukargewicht von weniger als 60000 Dalton und Polyvinylpyrrolidon. So wird in einer besonders bevorzugten Ausführungsform für die erfindungsgemäße Zubereitung eine Kombination aus mindestens zwei Cellulosederivaten bevorzugt, von denen mindestens eines wasserunlöslich ist, insbesondere eine Kombination aus Hydroxypropylcellulose und/oder Hydroxypropylmethylcellulose und wasserunlöslicher Ethylcellulose.

Die erfindungsgemäße Zubereitung kann mindestens ein Süßungsmittel, Geschmacksstoff, Konservierungsmittel, Farbstoff und/oder Füllstoff enthalten, wobei ein Gehalt von 0,1 bis 30 Gew.-%, weiter bevorzugt von 1 bis 15 Gew.-% bevorzugt wird, jeweils bezogen auf die getrocknete Zubereitung.

Die erfindungsgemäße Zubereitung kann z.B. eine Filmdicke von 1 bis 500 µm besitzen, bevorzugt 1 bis 300 µm.

Die erfindungsgemäße Zubereitung kann als runder, gerundeter, ovaler, ellipsenförmiger, dreieckiger, viereckiger oder vieleckiger Filmform vorliegen.

Ferner kann der erfindungsgemäße Film bzw. die erfindungsgemäße Zubereitung mit glatter Oberfläche oder Oberfläche mit Erhebungen und/oder Vertiefungen vorgesehen sein. Bevorzugt kann die Oberfläche ein regelmäßiges Muster aus Erhebungen und Vertiefungen aufweisen, wie beispielsweise ein Wellenmuster oder ein Gittermuster.

Ferner kann der erfindungsgemäße Film bzw. die erfindungsgemäße Zubereitung auf einer Trägerfolie vorgesehen sein.

Ferner kann der erfindungsgemäße Film bzw. die erfindungsgemäße Zubereitung mit einer Trägerfolie aus Polyethylen-Papier (PE-Papier), Polypropylen-Folie (PP-Folie) oder Polyethylenterephthalat-Folie (PET-Folie) vorgesehen werden. Bevorzugt wird der erfindungsgemäße Film bzw. die erfindungsgemäße Zubereitung auf einer Trägerfolie aus Polyethylen-Papier (PE-Papier), Polypropylen-Folie (PP-Folie) oder Polyethylenterephthalat-Folie (PET-Folie) versehen.

Der erfindungsgemäße Film bzw. die erfindungsgemäße zubereitung ist zu oraler Applikation vorgesehen. Ferner betrifft eine Ausführungsform der Erfindung einen Sachet-Beutel mit einer oder mehreren erfindungsgemäßen Filmen bzw. Zubereitungen.

Schließlich betrifft die Erfindung ein Mehrdosenbehältnis mit einer oder mehreren erfindungsgemäßen Filmen bzw. Zubereitungen.

Überraschenderweise wurde also gefunden, daß ein einschichtiger Film bzw. eine einschichtige Zubereitung gemäß den Ansprüchen eine deutlich höhere chemische Stabilität im Vergleich zu, beispielsweise Olanzapin-haltigen, Filmtabletten aufweist. Der Film haftet an der Mundhöhle und zerfällt innerhalb weniger Sekunden. Beispielsweise wird der Film durch Speichel aufgelöst oder zersetzt, z.B. wird ein wasserlöslicher Film aufgelöst. Somit ist der Film nicht mehr ausspuckbar. Nach Zerfall des Films wird der Wirkstoff überwiegend geschluckt und im gastrointestinalen Trakt resorbiert. Der Wirkstoff kann zum Teil transmucosal aufgenommen werden, dies ist aber vernachlässigbar. Der Film ist vorzugsweise im wesentlichen frei von Hohlräumen, Tensiden, Brausezusätzen oder Geschmacksmaskierern. Die Herstellung der Filme ist wesentlich kostengünstiger im Vergleich zu sogenannten Schmelztabletten, für deren Herstellung ein aufwendiger Lyophilisierungsprozeß erforderlich ist.

Bevorzugt umfasst die erfindungsgemäße Zubereitung mindestens zwei Filmbildner. Bevorzugt umfasst die erfindungsgemäße Zubereitung mindestens zwei Gelbildner. Besonders bevorzugt wird eine Kombination aus mindestens zwei Gelbildnern, wobei einer der Gelbildner bevorzugt wasserunlöslich ist.

In einer bevorzugten Ausführungsform umfasst die erfindungsgemäße Zubereitung ein oder mehrere Cellulosederivat(e) und ein synthetisches Polymer, insbesondere ein wasserunlösliches Cellulosederivat und ein wasserlösliches synthetisches Polymer. Bevorzugt umfasst die Zubereitung daneben einen oder mehrere weitere Filmbildner, ausgewählt aus der Gruppe, bestehend aus Sorbit, Polyethylenglykol, Polyethylenglykol-dioleat, 1,3-Butandiol, Propylenglykol, Isopropylpalmitat, Dibutylsebacat, Xylit und Paraffinöl. Bevorzugt umfasst die Zubereitung daneben einen oder mehrere weitere Gelbildner, insbesondere ein oder mehrere weitere Cellulosederivate, weiter bevorzugt ein oder mehrere Cellulosederivate mit einem Molekulargewicht von weniger als 60000 Dalton, und besonders bevorzugt Hydroxypropylcellulose und/oder Hydroxypropylmethylcellulose.

Eine solche Kombination aus mindestens einer wasserunlöslichen Verbindung und mindestens einer wasserlöslichen Verbindung führt dazu, dass die filmförmige Zubereitung vorteilhaft schnell den Wirkstoff freisetzt und gleichzeitig eine ausreichend hohe Stabilität aufweist.

In einer anderen bevorzugten Ausführungsform umfasst die erfindungsgemäße Zubereitung mehrere Cellulosederivate, von denen eines wasserunlöslich ist, insbesondere Hydroxypropylcellulose mit einem Molukargewicht von weniger als 60000 Dalton und/oder Hydroxypropylmethylcellulose mit einem Molukargewicht von weniger als 60000 Dalton und wasserunlösliche Ethylcellulose, und einen oder mehrere Verbindungen, ausgewählt aus der Gruppe, bestehend aus Sorbit, Polyethylenglykol, 1,3-Butandiol, Propylenglykol, Isopropylpalmitat, Dibutylsebacat, Xylit und Paraffinöl.

Filmbildner: Gelbildner können im Verhältnis von 0,7:10 bis 70:10, vorzugsweise 3:10 bis 50:10 vorliegen, insbesondere 4:10 bis 30:10, beispielsweise 5:10 bis 15:10. Besonders bevorzugt beträgt das Verhältnis Filmbildner: Gelbildner 5:10 bis 8:10.

Die Filme können als Wirkstoff neben Olanzapin einen oder mehrere Vertreter aus der Gruppe der Neuroleptika z.B. Benperidol, Haloperidol, Clozapin, Flupentixol, Fluphenazin, Droperidol, Melperon, Flupentixoldecanoat, Fluspirilen, Bromperidol, Pimozid, Trifluprometazin, Risperidon, Sertindol, Trifluperidol und/oder deren pharmazeutisch unbedenklichen Salze enthalten.

Der Wirkstoffgehalt im Film kann 0,1 bis 60 Gew.-% und insbesondere bis 50 Gew.-%, bevorzugt 25 Gew.-% betragen, jeweils bezogen auf die getrocknete Zubereitung.

Desweiteren kann der Film Süßungsmittel, Geschmacksstoffe, Konservierungsmittel (z.B. Sorbinsäure oder deren Salze), Farbstoffe und/oder Füllstoffe enthalten.

Als Süßungsmittel eignen sich Sucralose, Aspartam, Cyclamat, Saccharin und/oder Acesulfam, oder Kombinationen aus diesen Stoffen.

Als Geschmacksstoffe können natürliche oder künstliche Geschmacksstoffe, beispielsweise Zitronen-, Orangen-, Erdbeer-, Vanille-, Pfefferminzaroma, Cinnamylacetat, Citral, Citronella, Eugenylformat, Menthol und/oder Methylanisol verwendet werden. Als Farbstoffe können pharmazeutisch übliche Farbstoffe und Pigmente verwendet werden, insbesondere TiO₂, FeₓOₓ, β-Carotin, Azorubin, Indigotin, Riboflavin und dergleichen.

Als Füllstoffe können Salze wie Carbonate, Phophate, Oxide, wie z.B. SiO₂, insbesondere in Form von Aerosil, oder dergleichen und/oder Cellulose und ihre Derivate verwendet werden, aber auch schwerlösliche Zucker bzw. Zuckerderivate, wie zum Beispiel Lactose oder Stärkederivate wie Cyclodextrine, sofern diese im Produkt im wesentlichen ungelöst vorliegen und damit die mechanischen Eigenschaften eines Füllstoffs erfüllen. Bevorzugt wird SiO₂ als Füllstoff verwendet.

Die Dicke des Films kann 1 bis 500 µm betragen, vorzugsweise 1 bis 300 µm. Um ein unangenehmes Gefühl im Mund zu vermeiden, darf die Filmdicke nicht zu groß werden.

Die Filme können runde, ovale, ellipsenförmige, drei-, vier-oder vieleckige Formen aufweisen, sie können aber auch eine beliebig gerundete Form haben.

Die Oberfläche der Filme kann glatt sein oder mit Erhebungen oder Vertiefungen versehen sein.

Die Zerfallszeit der Filme in der Mundhöhle beträgt weniger als 200 Sekunden, bevorzugt 10 bis 60 Sekunden, insbesondere 10 bis 30 Sekunden.

Zur Herstellung des Films wird(werden) der(die) Wirkstoff(e) in einem Lösungsmittel suspendiert oder gelöst. Als Lösungsmittel können Alkohole oder Alkohol/Wasser-Gemische verwendet werden.

Nach Zugabe von Filmbildern, Gelbildern und ggf. Süßungsmitteln, Geschmacksstoffen, Farbstoffen und/oder Füllstoffen wird die Mischung homogenisiert. Die Mischung wird mit Hilfe eines geeigneten Streichverfahrens auf ein Trägermaterial aufgestrichen. Als Trägermaterial kann beispielsweise PE-Papier, PP- oder PET-Folie eingesetzt werden. Das beschichtete Trägermaterial wird bei 30 bis 120 °C, bevorzugt bei 30 bis 70°C getrocknet. Anschließend wird das beschichtete Trägermaterial zu flächenmäßig definierten, abgeteilten Filmen weiterverarbeitet. Dies kann durch Stanzung, Schneiden oder Prägung erfolgen. Die Filme werden einzeln mit oder ohne Trägerfolie in Sachet-Beutel verpackt. Sie können auch in Mehrdosenbehältnisse abgepackt werden. Vor der Einnahme wird der wirkstoffhaltige Film ggf. vom Trägermaterial abgezogen.

Die filmförmige Zubereitung wird erfindungsgemäß zur Verabreichung von Neuroleptika bei der Behandlung einer Störung im Zentralnervensystem, der Behandlung von Schizophrenie, der Behandlung einer schizophreniformen Krankheit, der Behandlung von akuter Manie, der Behandlung von leichten Angstzuständen und dergleichen verwendet. Bevorzugt wird die filmförmige Zubereitung zur Herstellung eines Arzneimittels für die Behandlung einer Störung im Zentralnervensystem, die Behandlung von Schizophrenie, die Behandlung einer schizophreniformen Krankheit, die Behandlung von akuter Manie, die Behandlung von leichten Angstzuständen und dergleichen eingesetzt.

Die Offenbarung wird durch nachstehende Beispiele näher erläutert, ohne aber den Erfindungsumfang damit einzuschränken. Sofern nicht anders angegeben beziehen sich alle Angaben in Gew.-% auf die getrocknete Zubereitung.

### Beispiel 1:

Die folgenden Stoffe werden zur Herstellung von Olanzapin-Filmen verwendet.

| **Bestandteile** | **Prozent** | **Gewicht** |
|---|---|---|
| Olanzapin | 50 | 50 |
| Hydroxypropylmethylcellulose | 30 | 30 |
| Ethylcellulose | 5 | 5 |
| Paraffinöl | 5 | 5 |
| D-Sorbit | 5 | 5 |
| 1,3-Butandiol | 2,5 | 2,5 |
| Isopropylpalmitat | 2,5 | 2,5 |
| Ethanol/Wasser | | 240* |

| | | |
|---|---|---|
| *wird während des Herstellprozesses entfernt | | |

### Herstellung:

Zur Herstellung des Films wird zunächst das D-Sorbit in Wasser gelöst. Zu dieser Lösung werden 1,3-Butandiol,
Isopropylpalmitat, Paraffinöl und Ethanol als Lösungsmittel zugegeben und gerührt. Dann werden zunächst die Ethylcellulose und die Hydroxypropylmethylcellulose zugegeben und gelöst und anschließend wird das Olanzapin zugewogen und die so erhaltene Suspension mit einem geeigneten Rührer homogenisiert.
Anschließend wird die Mischung unter Verwendung einer Beschichtungsmaschine auf einem geeigneten Träger z.B. PE-Folie ausgestrichen und bei 50°C das Ethanol/Wasser-Gemisch entfernt.

Der so erhaltene Film wird dann entsprechend der Dosierung ausgestanzt und verpackt.

### Vergleich der Stabilität des Olanzapinfilms mit filmüberzogener Olanzapin-Tablette

| Lagerdauer | Lagerbedingungen | Olanzapinfilm Verunreinigungen | Olanzapin-Filmtablette Verunreinigungen |
|---|---|---|---|
| 0 Monate | nicht kontrolliert | 0,03 | 0,37 |
| 0,5 Monate | 40°C/75% rF | 0,03 | |
| 3 Monate | 25°C/60% rF | | 0,43 |
| 3 Monate | 40°C/75% rF | | 0,73 |

Wie aus obiger Tabelle hervorgeht können in Olanzapin-haltigen Filmtabletten zum Teil bereits kurz nach der Herstellung nennenswerte Mengen an Verunreinigungen nachgewiesen werden, die bei weiterer Lagerung ansteigen. Im Vergleich dazu entstehen in der Filmzubereitung kaum nachweisbare Verunreinigungen.

### Beispiel 2:

| **Bestandteile** | **Prozent** | **Gewicht (g/100g)** |
|---|---|---|
| Olanzapin | 25 | 25 |
| Hydroxypropylcellulose | 15 | 15 |
| Polyvinylpyrrolidon | 37 | 37 |
| D-Sorbit | 10 | 10 |
| 1,3-Butandiol | 8 | 8 |
| Ethylcellulose | 5 | 5 |
| Ethanol/Wasser | | 240* |

| | | |
|---|---|---|
| *wird während des Herstellprozesses entfernt | | |

Die Herstellung erfolgt analog Beispiel 1.

### Beispiel 3:

Analog Beispiel 1 wurden Filme der in der folgenden Tabelle gezeigten Zusammensetzung mit unterschiedlichen Dosierungen an Olanzapin hergestellt.

| Bestandteil | Menge in mg pro Film | Menge in % |
|---|---|---|
| Olanzapin | 5, 10, 15, 20 | 25 |
| Hydroxypropylmethylcellulose | | 15 |
| Ethylcellulose | | 5 |
| Sorbitol | | 8,5 |
| Dibutylsebacat | | 5 |
| Isopropylpalmitat | | 3,5 |
| PEG | | 2 |
| Polyvinylpyrrolidon | | 25 |
| Aerosil | | 9 |
| Sucralose | | 1,5 |
| Orangen-Aroma | | 0,5 |

Die so hergestellten Filmzubereitungen zeigten mit Filmtabletten jeweils gleicher Dosierung vergleichbare Blutspiegel an Wirkstoff in Blutspiegelkurven.

## Patentansprüche

1. Filmförmige Zubereitung zur oralen Applikation umfassend
einen oder mehrere Filmbildner, ausgewählt aus der durch Ethylcellulose, Celluloseacetat, Cellulosephthalat, Sorbit, Xylit, Polyethylenglykol, 1,3-Butandiol, Propylenglykol, Isopropylpalmitat, Dibutylsebacat und Paraffinöl gebildeten Gruppe, wobei mindestens ein Filmbildner wasserunlöslich ist und ausgewählt ist aus der Gruppe wasserunlösliche Ethylcellulose, wasserunlösliches Celluloseacetat, wasserunlösliches Cellulosephthalat und Paraffinöl,
einen oder mehrere Gelbildner, wobei der Gelbildner ein Cellulosederivat mit einem Molekulargewicht von weniger als 60000 Dalton ist und Hydroxypropylcellulose und/oder Hydroxypropylmethylcellulose umfasst, und
Olanzapin.

2. Zubereitung gemäß Anspruch 1, wobei die Zubereitung ein fester Film ist.

3. Zubereitung gemäß Anspruch 1 oder 2, **dadurch gekennzeichnet, dass** sie einschichtig ist.

4. Zubereitung gemäß einem der Ansprüche 1 bis 3, **dadurch gekennzeichnet, dass** sie frei von Hohlräumen ist.

5. Zubereitung gemäß einem der Ansprüche 1 bis 4, **dadurch gekennzeichnet, dass** sie frei von Tensiden ist.

6. Zubereitung gemäß einem der Ansprüche 1 bis 5, **dadurch gekennzeichnet, dass** sie frei von Brausezusatz ist.

7. Zubereitung gemäß einem der Ansprüche 1 bis 6, **dadurch gekennzeichnet, dass** sie frei von Geschmacksmaskierern ist.

8. Zubereitung nach einem der Ansprüche 1 bis 7, wobei der Wirkstoffgehalt im Film 0,1 bis 60 Gew.-% und insbesondere bis 50 Gew.-% und vorzugsweise 20 bis 30 Gew.-% und besonders bevorzugt etwa 25 Gew.-% beträgt.

9. Zubereitung nach einem der vorstehenden Ansprüche, wobei der Gehalt des Films an Filmbildner 5 bis 70 Gew.-% beträgt.

10. Zubereitung nach einem der vorstehenden Ansprüche, wobei der Gehalt des Films an Gelbildner 10 bis 70 Gew.-% beträgt.

11. Zubereitung nach einem der vorstehenden Ansprüche, weiter umfassend ein Süßungsmittel, einen Geschmacksstoff, ein Konservierungsmittel, einen Farbstoff und/oder einen Füllstoff.

12. Zubereitung nach einem der vorstehenden Ansprüche, mit einer Filmdicke von 1 bis 500 µm.

13. Zubereitung nach einem der vorstehenden Ansprüche, mit runder, gerundeter, ovaler, ellipsenförmiger, dreieckiger, viereckiger oder vieleckiger Filmform.

14. Zubereitung nach einem der vorstehenden Ansprüche, mit glatter Oberfläche oder Oberfläche mit Erhebungen und/oder Vertiefungen .

15. Zubereitung nach einem der vorstehenden Ansprüche, wobei die Zubereitung auf einer Trägerfolie angeordnet ist.

16. Zubereitung nach Anspruch 15, wobei die Trägerfolie ausgewählt wird aus Polyethylen-Papier (PE-Papier), Polypropylen-Folie (PP-Folie) oder PolyethylenterephthalatFolie (PET-Folie) .

17. Sachet-Beutel mit einer oder mehreren Zubereitungen gemäß einem der Ansprüche 1 bis 16.

18. Mehrdosenbehältnis mit einer oder mehreren Zubereitungen gemäß mindestens einem der Ansprüche 1 bis 16.

19. Verfahren zur Herstellung einer Zubereitung gemäß einem der Ansprüche 1 bis 16, umfassend die Schritte: Auflösen des oder der Filmbildner in einem geeigneten Lösungsmittel, Zugeben des oder der Gelbildner, Zugeben des oder der Wirkstoffe, Homogenisieren der Mischung, Aufbringen der Mischung auf einen geeigneten Träger, und Entfernen des Lösungsmittels.

20. Zubereitung gemäß einem der Ansprüche 1 bis 16 zur Verwendung bei der Behandlung von einer Störung im Zentralnervensystem, zur Behandlung von Schizophrenie, zur Behandlung einer schizophreniformen Krankheit, zur Behandlung von akuter Manie und/oder zur Behandlung von leichten Angstzuständen.

21. Verwendung einer Zubereitung gemäß einem der Ansprüche 1 bis 16 zur Herstellung eines Arzneimittels zur Behandlung von einer Störung im Zentralnervensystem, zur Behandlung von Schizophrenie, zur Behandlung einer schizophreniformen Krankheit, zur Behandlung von akuter Manie und/oder zur Behandlung von leichten Angstzuständen.

## Claims

1. Film-form preparation for oral administration, comprising
one or more film former(s) selected from the group formed by ethylcellulose, cellulose acetate, cellulose phthalate, sorbitol, xylitol, polyethylene glycol, 1,3-butanediol, propylene glycol, isopropyl palmitate, dibutyl sebacate and paraffin oil, at least one film former being insoluble in water and being selected from the group: water-insoluble ethylcellulose, water-insoluble cellulose acetate, water-insoluble cellulose phthalate and paraffin oil,
one or more gel former(s), the gel former being a cellulose derivative having a molecular weight of less than 60,000 Dalton and comprising hydroxypropylcellulose and/or hydroxypropylmethylcellulose, and olanzapine.

2. Preparation according to claim 1, the preparation being a solid film.

3. Preparation according to claim 1 or 2, **characterised in that** it is single-layered.

4. Preparation according to any one of claims 1 to 3, **characterised in that** it is free of cavities.

5. Preparation according to any one of claims 1 to 4, **characterised in that** it is free of surfactants.

6. Preparation according to any one of claims 1 to 5, **characterised in that** it is free of effervescent additive.

7. Preparation according to any one of claims 1 to 6, **characterised in that** it is free of taste maskers.

8. Preparation according to any one of claims 1 to 7, the active ingredient content in the film being from 0.1 to 60 % by weight and especially up to 50 % by weight and preferably from 20 to 30 % by weight and more especially about 25 % by weight.

9. Preparation according to any one of the preceding claims, the film containing film former in an amount of from 5 to 70 % by weight.

10. Preparation according to any one of the preceding claims, the film containing gel former in an amount of from 10 to 70 % by weight.

11. Preparation according to any one of the preceding claims, further comprising a sweetener, a flavouring, a preservative, a colouring and/or a filler.

12. Preparation according to any one of the preceding claims, having a film thickness of from 1 to 500 µm.

13. Preparation according to any one of the preceding claims, having a round, rounded, oval, elliptical, triangular, quadrangular or polygonal film shape.

14. Preparation according to any one of the preceding claims, having a smooth surface or a surface having protuberances and/or depressions.

15. Preparation according to any one of the preceding claims, the preparation being arranged on a carrier foil.

16. Preparation according to claim 15, the carrier foil being selected from polyethylene paper (PE paper), polypropylene foil (PP foil) and poly-ethylene terephthalate foil (PET foil).

17. Sachet comprising one or more preparations according to any one of claims 1 to 16.

18. Multiple-dose container comprising one or more preparations according to at least one of claims 1 to 16.

19. Process for the production of a preparation according to any one of claims 1 to 16, comprising the steps of: dissolving the film former(s) in a suitable solvent, adding the gel former(s), adding the active ingredient(s), homogenising the mixture, applying the mixture to a suitable carrier, and removing the solvent.

20. Preparation according to any one of claims 1 to 16 for use in the treatment of a disorder in the central nervous system, in the treatment of schizophrenia, in the treatment of a schizophreniform disease, in the treatment of acute mania and/or in the treatment of mild anxiety states.

21. Use of a preparation according to any one of claims 1 to 16 in the production of a medicament for the treatment of a disorder in the central nervous system, the treatment of schizophrenia, the treatment of a schizophreniform disease, the treatment of acute mania and/or the treatment of mild anxiety states.

## Revendications

1. Préparation en forme de film pour une application orale comprenant
un ou plusieurs agents filmogènes choisis dans le groupe formé par l'éthylcellulose, l'acétate de cellulose, le phtalate de cellulose, le sorbitol, le xylitol, le polyéthylèneglycol, le 1,3-butanediol, le propylèneglycol, le palmitate d'isopropyle, le sébacate de dibutyle et l'huile de paraffine, au moins un agent filmogène étant insoluble dans l'eau et étant choisi dans le groupe des éthylcelluloses insolubles dans l'eau, de l'acétate de cellulose insoluble dans l'eau, du phtalate de cellulose insoluble dans l'eau et de l'huile de paraffine,
un ou plusieurs agents gélifiants, l'agent gélifiant étant un dérivé de cellulose avec une masse moléculaire inférieure à 60000 Dalton et comprenant de l'hydroxypropylcellulose et/ou de l'hydroxypropylméthylcellulose, et
de l'olanzapine.

2. Préparation selon la revendication 1, la préparation étant un film solide.

3. Préparation selon les revendications 1 ou 2, **caractérisée en ce qu'**elle est monocouche.

4. Préparation selon l'une des revendications 1 à 3, **caractérisée en ce qu'**elle est exempte de cavités.

5. Préparation selon l'une des revendications 1 à 4, **caractérisée en ce qu'**elle est exempte de tensioactifs.

6. Préparation selon l'une des revendications 1 à 5, **caractérisée en ce qu'**elle est exempte d'additif effervescent.

7. Préparation selon l'une des revendications 1 à 6, **caractérisée en ce qu'**elle est exempte d'agents masquant le goût.

8. Préparation selon l'une des revendications 1 à 7, la teneur en matière active dans le film s'élevant de 0,1 à 60 % en poids et notamment jusqu'à 50 % en poids, et de préférence de 20 à 30 % en poids, et de manière particulièrement préférée, à environ 25 % en poids.

9. Préparation selon l'une des revendications précédentes, la teneur en agent filmogène du film s'élevant de 5 à 70 % en poids.

10. Préparation selon l'une des revendications précédentes, la teneur en agent gélifiant du film s'élevant de 10 à 70 % en poids.

11. Préparation selon l'une des revendications précédentes, comprenant en outre un agent édulcorant, un agent aromatisant, un agent de conservation, un colorant et/ou une charge.

12. Préparation selon l'une des revendications précédentes, avec une épaisseur de film de 1 à 500 µm.

13. Préparation selon l'une des revendications précédentes, avec une forme de film ronde, arrondie, ovale, elliptique, triangulaire, carrée ou polygonale.

14. Préparation selon l'une des revendications précédentes, avec une surface lisse ou une surface comprenant des élévations et/ou des creux.

15. Préparation selon l'une des revendications précédentes, la préparation étant disposée sur une feuille de support.

16. Préparation selon la revendication 15, la feuille de support étant choisie parmi du papier-polyéthylène (papier-PE), une feuille de polypropylène (feuille PP) ou une feuille de polyéthylène téréphtalate (feuille PET).

17. Sachet-pochon comprenant une ou plusieurs préparations selon l'une des revendications 1 à 16.

18. Conditionnement multi-doses comprenant une ou plusieurs préparations selon au moins l'une des revendications 1 à 16.

19. Procédé de fabrication d'une préparation selon l'une des revendications 1 à 16 comprenant les étapes de : dissolution de ou des agents filmogènes dans un solvant approprié, ajout du ou des agents gélifiants, ajout de la ou des matières actives, homogénéisation du mélange, mise en place du mélange sur un support approprié, et enlèvement du solvant.

20. Préparation selon l'une des revendications 1 à 16 pour une utilisation dans le traitement d'un trouble dans le système nerveux central, pour le traitement de la schizophrénie, pour le traitement d'une maladie schizophréniforme, pour le traitement d'une manie aiguë et/ou pour le traitement d'états d'angoisse légère.

21. Utilisation d'une préparation selon l'une des revendications 1 à 16 pour la fabrication d'un produit pharmaceutique destiné au traitement d'un trouble dans le système nerveux central, pour le traitement de la schizophrénie, pour le traitement d'une maladie schizophréniforme, pour le traitement d'une manie aiguë et/ou pour le traitement d'états d'angoisse légère.
